# EUROPÄISCHE PATENTANMELDUNG

(11) **EP 4 624 134 A1**
(43) Veröffentlichungstag der Anmeldung: **01.10.2025**
(21) Anmeldenummer: 24166677.5
(22) Anmeldetag: 27.03.2024
(51) Int. Cl.: B29C 57/00

(54) **VORRICHTUNG UND VERFAHREN ZUM FORMEN DES FREIEN ENDES EINES SCHLAUCHABSCHNITTS**

(71) Anmelder: Harro Höfliger Verpackungsmaschinen GmbH, 71573 Allmersbach im Tal (DE)
(72) Erfinder: Schaal, Florian, 71573 Allmersbach im Tal (DE); Bantel, Holger, 71573 Allmersbach im Tal (DE); Stahl, Ulrich, 71573 Allmersbach im Tal (DE); Pohl, Bernd, 71573 Allmersbach im Tal (DE); Marbaz, Jens, 71573 Allmersbach im Tal (DE)
(74) Vertreter: Schmid, Barbara

(57) **Zusammenfassung**

Die Erfindung betrifft eine Vorrichtung und ein Verfahren zum Formen des freien Endes (12) eines Schlauchabschnitts. Die Vorrichtung besitzt eine Zuführeinheit und eine Transporteinheit für den Schlauchabschnitt sowie zumindest eine Bearbeitungsstation (50) für das freie Ende (12) des Schlauchabschnitts. Erfindungsgemäß weist die Bearbeitungsstation (50) einen Werkzeugkern (58) auf, über den das freie Ende (12) des Schlauchabschnitts geschoben werden kann. Darüber hinaus weist die Bearbeitungsstation (50) ein Formwerkzeug (52) auf, das von außen um das freie Ende (12) des Schlauchabschnitts herum positioniert werden kann, so dass die Außenkontur des freien Endes (12) des Schlauchabschnitts durch das Formwerkzeug (52) verformt werden kann. Mittels eines Heizsystems (60) kann das freie Ende (12) des Schlauchabschnitts und/oder das Formwerkzeug (52) erwärmt werden. Bei dem erfindungsgemäßen Verfahren wird zunächst das freie Ende (12) des Schlauchabschnitts über eine Linearbewegung in eine Bearbeitungsstation (50) eingeführt. Dabei wird das freie Ende (12) des Schlauchabschnitts über einen Werkzeugkern (58) geführt. Anschließend wird das freie Ende (12) des Schlauchabschnitts und/oder das Formwerkzeug (52) der Bearbeitungsstation (50) erhitzt und über eine Linearbewegung weiter in das Formwerkzeug (52) eingefahren, so dass eine Umformung der Außenkontur des freien Endes (12) des Schlauchabschnitts erfolgt.

## Beschreibung

### TECHNISCHES GEBIET

Die Erfindung betrifft eine Vorrichtung und ein Verfahren zum Formen des freien Endes eines Schlauchabschnitts. Das Formen des freien Endes eines Schlauchabschnitts kann insbesondere zur Herstellung einer Kunststoff-Kanüle mit einer Spitze und einem Kopf erforderlich sein. Derartige Kunststoff-Kanülen können beispielsweise bei der subkutanen Medikamentengabe eingesetzt werden. Das Formen des freien Endes eines Schlauchabschnitts kann darüber hinaus auch für weitere medizinische Anwendungen, beispielsweise für Urinkatheter, für Coronare Herzkatheter, für zentralvenöse Katheter oder für unterschiedliche Absaugkatheder erforderlich sein.

### STAND DER TECHNIK

Die Bearbeitung von Schlauchabschnitten oder anderen biegeschlaffen Teilen erfolgt in der Regel in mehreren Bewegungsschritten. Regelmäßig werden die Schläuche zunächst zugeführt abgelängt und für die Weiterverarbeitung aufgenommen und ausgerichtet. Alternativ können auch bereits abgelängte Schlauchstücke oder Halbzeuge verwendet werden. Das Aufnehmen und Ausrichten der Schlauchabschnitte kann dabei insbesondere mittels Greifer erfolgen. Aus der EP 3 456 529 B1 ist eine Vorrichtung und ein Verfahren bekannt, bei der die Bearbeitung der Schlauchabschnitte während des Transports derselben erfolgen kann, so dass die Schlauchabschnitt kontinuierlich bearbeitet werden können, ohne diese aus ihrer Orientierung entlassen zu müssen.

Es ist bekannt, das freie Ende eines Schlauchabschnitts in einem entsprechenden Formatwerkzeug thermisch zu verformen. Abhängig von der gewünschten Außenkontur des freien Endes des Schlauchabschnitts kann es dabei gegebenenfalls erforderlich sein, beispielsweise einen Kopf separat zu formen und anschließend anzugießen.

### DARSTELLUNG DER ERFINDUNG

Ausgehend von diesem vorbekannten Stand der Technik liegt der Erfindung die Aufgabe zugrunde, eine verbesserte Vorrichtung und ein verbessertes Verfahren zum Formen des freien Endes eines Schlauchabschnitts anzugeben, so dass ein vollautomatisierter Produktionsprozess möglich ist, bei dem besonders exakte Ergebnisse möglich sind.

Die erfindungsgemäße Vorrichtung zum Formen des freien Endes eines Schlauchabschnitts ist durch die Merkmale des Hauptanspruchs 1 gegeben. Das erfindungsgemäße Verfahren zum Formen des freien Endes eines Schlauchabschnitts ist durch die Merkmale des nebengeordneten Anspruchs 8 gegeben. Sinnvolle Weiterbildungen der Erfindung sind Gegenstand von sich an diese Ansprüche anschließenden weiteren Ansprüchen.

Die erfindungsgemäße Vorrichtung zum Formen des freien Endes eines Schlauchabschnitts besitzt eine Zuführeinheit für den Schlauchabschnitt, eine Transporteinheit für den Schlauchabschnitt und zumindest eine Bearbeitungsstation für das freie Ende des Schlauchabschnitts. Erfindungsgemäß weist die Bearbeitungsstation einen Werkzeugkern auf. Über diesen Werkzeugkern wird das freie Ende des Schlauchabschnitts geführt. Dadurch bleibt ein offener Innendurchmesser des Schlauchabschnitts bestehen. Die Bearbeitungsstation weist ein Formwerkzeug auf, das von außen um das freie Ende des Schlauchabschnitts herum positionierbar ist, so dass die Außenkontur des freien Endes des Schlauchabschnitts durch das Formwerkzeug verformbar ist. Darüber hinaus weist die Bearbeitungsstation ein Heizsystem auf, mittels dem das freie Ende des Schlauchabschnitts und/oder das Formwerkzeug erwärmt werden kann, so dass dieses freie Ende des Schlauchabschnitts verformt werden kann.

Die Außenkontur des freien Endes des Schlauchabschnitts nach dem Verformen wird somit von der Innenkontur des Formwerkzeuges bestimmt. Die Qualität der Innenkontur des Formwerkzeugs bestimmt somit die Qualität der Außenkontur des verformten Schlauchendes. Der Innendurchmesser des verformten Schlauchendes wird dagegen von der Außenkontur des Werkzeugkerns bestimmt. Der Werkzeugkern kann somit vorzugsweise austauschbar ausgebildet sein, so dass eine einfache Anpassung an unterschiedliche Innendurchmesser der Schlauchabschnitte möglich ist. Vorzugsweise können der Werkzeugkern und das Formwerkzeug separat voneinander ausgetauscht werden, so dass eine möglichst große Variabilität hinsichtlich der möglichen Konturen des verformten Endes des Schlauchabschnitts besteht. Durch den Einsatz von Präzisionswerkzeugen kann daher eine sehr gute Oberflächengüte der verformten Schlauchenden erreicht werden.

Die Werkzeuge der Bearbeitungsstation, insbesondere das Formwerkzeug und der Werkzeugkern sind verschmutzungs- und verschleißarme Werkzeuge mit einer hohen Standzeit. In der Regel kommt der Umformprozess ohne die Zugabe von Zusatzstoffen aus, so dass die erfindungsgemäße Vorrichtung auch unter Reinraum-Bedingungen betrieben werden kann.

Die Transporteinheit der erfindungsgemäßen Vorrichtung kann vorzugsweise eine Linearbewegung des Schlauchabschnitts innerhalb der Bearbeitungsstation, insbesondere innerhalb des Formwerkzeugs, ermöglichen. Das freie Ende des Schlauchabschnitts kann somit während des Formprozesses innerhalb des Formwerkzeugs verschoben werden, so dass beispielsweise auch ein Umformen des freien Endes des Schlauchabschnitts nach innen oder nach außen möglich ist. Auf diese Weise kann beispielsweise ein Flansch direkt aus dem freien Ende des Schlauchabschnitts geformt werden.

Die Transporteinheit kann in diesem Fall eine Greifereinheit aufweisen, durch die der zu bearbeitende Schlauchabschnitt fixiert werden kann. Die Greifereinheit kann auf einer Lineareinheit befestigt sein, so dass die Greifereinheit in einer Linearbewegung hin und her verfahren werden kann. Bei einer solchen Lineareinheit kann es sich beispielsweise um einen auf einer Schiene gelagerten Linearschlitten handeln.

Die Zuführeinheit kann grundsätzlich variieren, abhängig von der Art der zugeführten Schlauchabschnitte. In einer ersten Ausführungsform können die Schlauchabschnitte als Endlosmaterial von einer Materialrolle abgewickelt werden. Die Zuführeinheit kann in diesem Fall zumindest eine Greifereinheit aufweisen, durch die der Endlosschlauch nach und nach von der Materialrolle abgewickelt werden kann. In diesem Fall sollte die Zuführeinheit eine Schneideinheit aufweisen, mittels der der Endlosschlauch in die Schlauchabschnitte in der gewünschten Länge geschnitten werden kann. Gegebenenfalls kann in diesem Fall eine vorgelagerte Bearbeitungsstation vorhanden sein, in der der Endlosschlauch geradegerichtet wird. Dies kann beispielsweise durch ein Richten des Endlosschlauchs erfolgen.

Alternativ dazu können die Schlauchabschnitte auch einzeln oder als Schüttware zugeführt werden. In diesem Fall können die Schlauchabschnitt beispielsweise manuell an der Transporteinheit befestigt werden. Die Zuführeinheit könnte in diesem Fall auch eine Vereinzelungseinheit aufweisen, durch die die Schlauchabschnitt zunächst vereinzelt und ausgerichtet werden, damit diese automatisiert an die Transporteinheit übergeben werden können.

Bevor die Schlauchabschnitte der Bearbeitungsstation und damit dem Formwerkzeug zugeführt werden, können die Schlauchabschnitte zunächst zumindest einer vorgelagerten Bearbeitungsstation zugeführt werden. Bei einer solchen vorgelagerten Bearbeitungsstation kann es sich beispielsweise um eine Wärmebehandlungseinheit und/oder eine Ionisierungseinheit handeln. Eine solche vorgelagerte Bearbeitungsstation kann insbesondere dazu dienen, den von der Materialrolle abgewickelten Endlosschlauch geradezurichten. Alternativ oder zusätzlich kann zumindest eine vorgelagerte Bearbeitungsstation eine Gebläseeinheit aufweisen, um mögliche Partikel von den zu verformenden Schlauchabschnitten abzublasen.

Vorzugsweise kann zumindest eine Sensoreinheit vorhanden sein, mittels der eine Überprüfung des Schlauchabschnitts durchgeführt werden kann. Auf diese Weise kann der Schlauchabschnitt beispielweise vor der Umformung auf Beschädigungen überprüft werden. Auch eine Kontrolle der zu verformenden Schlauchabschnitte, insbesondere auf Unregelmäßigkeiten hinsichtlich der Innen- und Außendurchmesser der Schlauchabschnitte, kann mittels einer solchen Sensoreinheit durchgeführt werden. Auch die verformten Schlauchabschnitte können mit einer solchen Sensoreinheit überprüft werden. Auf diese Weise ist eine vollautomatisierte Qualitätskontrolle möglich.

Das Heizsystem kann vorzugsweise nach dem Widerstandsheizverfahren arbeiten. Dabei können die Heizprozessparameter, beispielweise während der Aufheizphase oder der Abkühlphase, frei eingestellt werden. Abhängig von der Geometrie des Formwerkzeugs kann die Erhitzungszone im Formwerkzeug variiert werden. In der Regel erfolgt die Erhitzung im Einstichbereich des Formwerkzeugs, also an der Stelle mit dem kleinsten Querschnitt. Wird die Stelle des kleinsten Querschnitts an einen anderen Bereich des Formwerkzeugs verlegt, kann somit auch die Erhitzungszone verlegt werden.

Die Bearbeitungsstation kann vorzugsweise eine Kühlung aufweisen. Mittels dieser Kühlung kann das Abkühlen des verformten Schlauchendes besonders rasch erfolgen, so dass besonders kurze Prozesszeiten erreicht werden können.

Das Formwerkzeug kann in einer bevorzugten Ausführungsform zumindest zwei Formteile aufweisen. Die Formteile können in einer definierten Bewegung aufeinander zu und voneinander wegbewegt werden, so dass das Formwerkzeug geöffnet und geschlossen werden kann. Dies erleichtert das Entnehmen des verformten Schlauchendes, insbesondere bei einem nach außen oder nach innen verformten Schlauchende. Der nach außen und/oder innen verformte Bereich kann somit nicht weiter verformt und damit beschädigt werden. Das Öffnen des Formwerkzeugs kann dabei in radialer Richtung oder in Längsrichtung erfolgen, abhängig von der Konstruktion der einzelnen Formteile. Bei einer radialen Öffnung des Formwerkzeugs können die einzelnen Formteile beispielsweise jeweils in Art einer Halbschale ausgebildet sein und entsprechend radial auseinander bewegt werden. Bei einer Öffnung des Formwerkzeugs in Längsrichtung können die Formteile dagegen als ineinander schiebbare Hülsen ausgebildet sein.

Die erfindungsgemäße Vorrichtung kann vorzugsweise über eine Entnahmevorrichtung verfügen. Mittels der Entnahmevorrichtung kann der Schlauchabschnitt mit dem verformten Ende aus der Transporteinheit entnommen werden. Anschließend kann der Schlauchabschnitt noch abgelängt werden, sofern es sich um einen Endlosschlauch handelt. Der zugeschnittene Schlauchabschnitt kann durch die Entnahmevorrichtung anschließend an eine weitere Station übergeben werden, um den Schlauchabschnitt weiter zu bearbeiten. Bei einer solchen weiteren Station kann es sich beispielsweise auch um eine weitere erfindungsgemäße Vorrichtung handeln, in der dann das gegenüberliegende freie Ende des Schlauchabschnitts verformt werden kann. Bei einer solchen Entnahmevorrichtung kann es sich beispielsweise um einen Roboter oder einen XY-Greifer handeln.

Bei dem erfindungsgemäßen Verfahren zum Formen des freien Endes eines Schlauchabschnitts wird zunächst das freie Ende des Schlauchabschnitts über eine Linearbewegung in eine Bearbeitungsstation eingeführt. Dabei wird das freie Ende des Schlauchabschnitts über einen Werkzeugkern der Bearbeitungsstation geführt. Das freie Ende des Schlauchabschnitts und/oder das Formwerkzeug der Bearbeitungsstation wird erhitzt. Nach dem Erreichen der gewünschten Temperatur wird das freie Ende des Schlauchabschnitts über eine Linearbewegung weiter in das Formwerkzeug eingefahren, so dass eine Umformung der Außenkontur des freien Endes des Schlauchabschnitts erfolgen kann.

Durch das Aufschieben des freien Endes des Schlauchabschnitts auf den Werkzeugkern kann verhindert werden, dass der Schlauchinnendurchmesser zugeschmolzen wird.

Vorzugsweise kann der Schlauchabschnitt vor dem Einführen in die Bearbeitungsstation mittels einer Sensoreinheit überprüft werden. Dabei kann insbesondere eine Überprüfung auf mögliche Beschädigungen oder auf Klebestellen erfolgen. Auch eine Überprüfung der Außen- und Innendurchmesser der Schlauchabschnitte kann mittels der Sensoreinheit erfolgen. Sofern der zu formende Schlauchabschnitt bei der Überprüfung als fehlerhaft erkannt wird, kann der Schlauchabschnitt noch vor der eigentlichen Bearbeitung ausgeschleust werden.

Alternativ oder zusätzlich dazu kann der Schlauchabschnitt vor dem Einführen in die Bearbeitungsstation mittels einer vorgelagerten Bearbeitungsstation vorbehandelt werden. Dabei kann der Schlauchabschnitt beispielsweise leicht erwärmt werden, um einen Endlosschlauch geradezurichten. Auch eine Ionisierung kann in diesem Zusammenhang durchgeführt werden. Darüber hinaus könnte auch ein Abblasen von möglicherweise außen anhaftenden Partikeln von dem Schlauchabschnitt erfolgen.

Das freie Ende des Schlauchabschnitts wird so weit in die Bearbeitungsstation mit dem Formwerkzeug eingeführt, bis eine vorbestimmte Position erreicht wird. Diese vorbestimmte Position kann durch den verfahrenen Weg definiert werden; alternativ dazu kann die vorbestimmte Position auch durch die für das Aufschieben des freien Endes des Schlauchabschnitts auf den Werkzeugkern benötigte Kraft definiert werden. Die jeweiligen Prozessparameter für das Einführen des Schlauchabschnitts in die Bearbeitungsstation mit dem Formwerkzeug sind dabei grundsätzlich frei einstellbar. Bei diesen Prozessparametern kann es sich beispielsweise um die Geschwindigkeit beim Einfahren, um die Beschleunigung beim Beginn des Einfahr-Vorgangs, um die Verzögerung zum Abschluss des Einfahr-Vorgangs, und/oder um die definierte Endstellung handeln.

Die Prozessparameter zum Heizen des Formwerkzeugs können grundsätzlich frei eingestellt werden.

Nach dem Aktivieren des Heizsystems wird das freie Ende des Schlauchabschnitts weiter in das Formwerkzeug eingeschoben. Dabei erfolgt das Umformen des freien Endes des Schlauchabschnitts, indem beispielsweise die Spitze oder der Kopf geformt werden. Abhängig vom Material des Schlauchabschnitts und der Art der Umformung kann das Ende des Umform-Prozesses durch den verfahrenen Weg innerhalb des Formwerkzeugs und/oder durch die aufgebrachte Kraft zum Verfahren innerhalb des Formwerkzeugs und/oder durch die Zeit für das Verfahren innerhalb des Formwerkzeugs bestimmt werden.

Nach Beendigung des Umform-Prozesses wird das Erhitzen des Formwerkzeugs der Bearbeitungsstation beendet, indem das entsprechende Heizsystem abgeschaltet wird. Das freie Ende des Schlauchabschnitts kann jedoch noch für eine gewisse Zeit in der eingefahrenen Position verbleiben. Dies führt zu besonders exakten Umform-Ergebnissen und minimiert das Risiko von nachträglichen unerwünschten Verformungen.

Zum schnelleren Abkühlen des Formwerkzeugs kann nach dem Abschalten des Heizsystems eine Kühlung aktiviert werden. Die Kühlung kann nach dem Unterschreiten einer einstellbaren Temperaturschwelle und/oder nach Ablauf einer gewissen Zeitspanne beendet werden.

In einer vorteilhaften Ausführungsform kann das Formwerkzeug zumindest zwei Formteile aufweisen, so dass das Formwerkzeug geöffnet und geschlossen werden kann. Das Formwerkzeug sollte in diesem Fall vor dem weiteren Einfahren des freien Endes des Schlauchabschnitts geschlossen sein. Nach dem Umformen der Außenkontur des freien Endes des Schlauchabschnitts kann das Formwerkzeug wieder geöffnet werden, um das Entnehmen des verformten Schlauchabschnitts zu erleichtern. Das Öffnen des Formwerkzeugs sollte dabei erst nach dem Abkühlen des Formwerkzeugs erfolgen.

Das Öffnen des Formwerkzeugs kann dabei insbesondere pneumatisch oder elektrisch erfolgen. Vorzugsweise kann eine der Formteile in ihrer Position verbleiben, so dass das verformte Ende des Schlauchabschnitts durch dieses Formteil weiter gestützt und stabilisiert werden kann. Die übrigen Formteile können dann von diesem stationären Formteil entfernt werden.

Um den verformten Schlauchabschnitt leichter aus dem Formwerkezug lösen zu können, kann das Formwerkzeug während des Öffnens über einen definierten und geregelten Heizprozess nochmals kurz erwärmt werden.

Um den verformten Schlauchabschnitt weiter bearbeiten zu können, kann das verformte freie Ende des Schlauchabschnitts über eine Linearbewegung aus dem Formwerkzeug herausgefahren werden. Das freie Ende des Schlauchabschnitts ist dadurch gut zugänglich und kann von einer Entnahmevorrichtung entnommen und beispielsweise an eine weitere Bearbeitungsstation übergeben werden.

Ein solches erfindungsgemäßes Verfahren kann grundsätzlich vollautomatisch, halbautomatisch oder manuell durchgeführt werden. Bei einem vollautomatischen Prozess erfolgt sowohl die Zuführung des Schlauchabschnitts als auch die Entnahme des verformten Schlauchabschnitts automatisiert. In diesem Fall kann vor der eigentlichen Entnahme des verformten Schlauchabschnitts noch ein Ablängen des Schlauchabschnitts erfolgen. Die Schlauchabschnitt können in diesem Fall als Endlosschlauch zugeführt werden, wobei die Bearbeitung und Verformung des freien Endes noch vor dem eigentlichen Zuschneiden des Schlauchabschnitts erfolgen kann. Wird das freie Ende des Schlauchs nach dem Abschluss des Umformprozesses von der Transporteinheit oder der Entnahmevorrichtung fixiert, kann das Ablängen des Schlauchabschnitts erfolgen, wobei die Länge des Schlauchabschnitts grundsätzlich frei wählbar ist. Bei einem halbautomatischen Prozess kann beispielsweise die Zuführung der Schlauchabschnitte automatisiert erfolgen (beispielsweise auch von einem Endlosschlauch), während die Entnahme des verformten Schlauchabschnitts manuell erfolgt. Alternativ dazu kann auch die Zuführung der Schlauchabschnitte manuell erfolgen, indem diese einzeln an der Transporteinrichtung fixiert werden. In diesem Fall können die Schlauchabschnitte beispielsweise als Schuttgut vorgelegt werden.

Die Anforderungen an die Formqualität und die Einhaltung exakter Maße bei den fertig verformten Schlauchabschnitten sind in der Regel sehr hoch. Der Umformprozess reagiert dabei sehr empfindlich auf Veränderungen der einzelnen Prozessparameter, beispielsweise hinsichtlich des Materials des Schlauchabschnitts, der Temperatur, der Umformzeiten, der Geschwindigkeiten der Linearbewegungen, der Formwerkzeuge inklusive der Werkzeugkerne und der Geometrien der verformten Schlauchabschnitte.

Mittels einer solchen Vorrichtung beziehungsweise einem solchen Verfahren können grundsätzlich beide Enden eines Schlauchabschnitts bearbeitet und verformt werden. Dadurch kann beispielsweise das spätere vordere Ende des Schlauchabschnitts zu einer Spitze verformt werden, während das spätere hintere Ende des Schlauchabschnitts zu einem Kopf verformt werden kann. Alternativ dazu könnten auch beide Enden des Schlauchabschnitts jeweils zu einer Spitze verformt werden, wobei die beiden Spitzen der in diesem Fall identisch oder unterschiedlich geformt sein können. Entsprechend könnten auch beide Enden des Schlauchabschnitts zu identischen oder unterschiedlich geformten Köpfen verformt werden. Darüber hinaus wäre es auch möglich, lediglich eines der beiden Enden eines Schlauchabschnitts zu bearbeiten, sofern lediglich eines der beiden Enden entsprechend verformt werden muss.

Die Form und die Geometrie (Innen- und Außengeometrie) der Spitze und/oder des Kopfes ist grundsätzlich frei wählbar. Auch hinsichtlich der Länge des Schlauchabschnitts sind bei der Vorrichtung und dem Verfahren keine Einschränkungen gegeben.

Mit dem erfindungsgemäßen Verfahren ist ein geregelter Formprozess gegeben, der zu reproduzierbaren Ergebnissen führt. Der Formprozess ist dabei positions- und/oder kraftgeregelt, so dass eine Anpassung an unterschiedliche Anforderungen einfach möglich ist.

Mit der erfindungsgemäßen Vorrichtung beziehungsweise dem erfindungsgemäßen Verfahren können Schlauchabschnitte aus allen Materialien bearbeitet und umgeformt werden, die grundsätzlich umformbar sind.

Weitere Vorteile und Merkmale der Erfindung sind den in den Ansprüchen ferner angegebenen Merkmalen sowie den nachstehenden Ausführungsbeispielen zu entnehmen.

### KURZE BESCHREIBUNG DER ZEICHNUNG

Die Erfindung wird im Folgenden anhand der in der Zeichnung dargestellten Ausführungsbeispiele näher beschreiben und erläutert. Es zeigen:
- Fig. 1: eine schematische Ansicht einer ersten Ausführungsform der erfindungsgemäßen Vorrichtung zum Formen des freien Endes eines Schlauchabschnitts,
- Fig. 2: eine schematische Ansicht der Bearbeitungsstation der erfindungsgemäßen Vorrichtung gemäß Fig 1,
- Fig. 3: ein schematischer Längsschnitt durch das Formwerkzeug und die Werkzeugspitze der Bearbeitungsstation gemäß Fig. 2 und
- Fig. 4: ein schematischer Längsschnitt durch das Formwerkzeug und die Werkzeugspitze der Bearbeitungsstation einer zweiten Ausführungsform der erfindungsgemäßen Vorrichtung.

### WEGE ZUM AUSFÜHREN DER ERFINDUNG

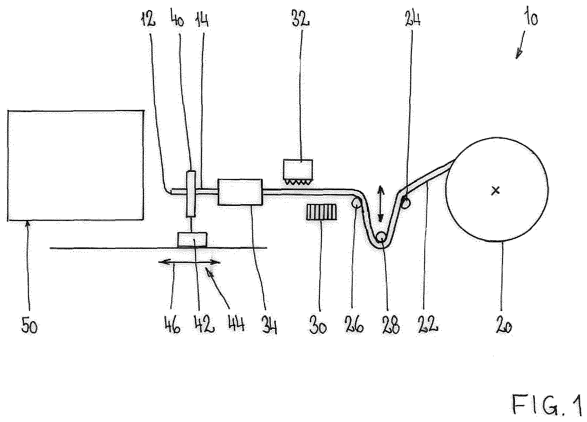

Eine erste Ausführungsform der erfindungsgemäßen Vorrichtung 10 zum Formen des freien Endes 12 eines Schlauchabschnitts 14 ist schematisch in Fig. 1 dargestellt.

Die Vorrichtung 10 besitzt im vorliegenden Beispielsfall eine Materialrolle 20, auf der ein Endlosschlauch 22 aufgewickelt vorhanden ist. Das freie Ende 12 des Endlosschlauchs 22 wird im vorliegenden Beispielsfall über zwei stationäre Rollen 24, 26 sowie über eine bewegliche Rolle 28 geführt. Die bewegliche Rolle 28 ist dabei zwischen den beiden stationären Rollen 24, 26 angeordnet.

Anschließend wird das freie Ende 12 durch zwei vorgelagerte Bearbeitungsstationen 30, 32 geführt. In der vorgelagerten Bearbeitungsstation 30 wird der Endlosschlauch 22 mit Wärme behandelt; in der vorgelagerten Bearbeitungsstation 32 erfolgt eine Ionisierung des Endlosschlauchs 22. Durch die beiden vorgelagerten Bearbeitungsstationen 30, 32 soll die Krümmung des Endlosschlauchs 22 reduziert werden, so dass ein möglichst gerade gerichteter Endlosschlauch 22 erhalten wird. Abhängig vom Material des Endlosschlauchs 22 und den gewählten Prozessparametern kann auf die Bearbeitungsstationen 30, 32 auch verzichtet werden.

Darüber hinaus wäre es auch möglich, weitere vorgelagerte Bearbeitungsstationen vorzusehen, in denen beispielsweise ein Abblasen möglicher Partikel von dem Endlosschlauch 22 erfolgen kann.

Vor oder nach den Bearbeitungsstationen 30, 32 könnte eine Sensoreinheit eingesetzt werden, die beispielsweise über ein Kamerasystem verfügen kann. Dadurch kann der Endlosschlauch 22 auf Beschädigungen und Klebestellen überprüft werden. Auch eine Kontrolle der Außen- und Innendurchmesser des Endlosschlauchs 22 wäre auf diese Weise möglich.

Anschließend an die Bearbeitungsstationen 30, 32 ist im vorliegenden Beispielsfall eine stationäre Greifereinheit 34 angeordnet. Die Greifereinheit 34 wird nicht zum Abwickeln des Endlosschlauchs 22 von der Materialrolle 20 benötigt, so dass die Greifereinheit 34 während des Abwickelns des Endlosschlauchs 22 geöffnet ist. Das freie Ende 12 des Endlosschlauchs 22 passiert diese stationäre Greifereinheit daher zunächst lediglich. Hinter der stationären Greifereinheit 34 ist eine weitere Greifereinheit 40 vorhanden. Die Greifereinheit 40 ist auf einer Lineareinheit 42 befestigt. Die Greifereinheit 40 ist somit linearverschieblich und bildet gemeinsam mit der Lineareinheit 42 eine Transporteinheit 44 für den Endlosschlauch 22. Mittels der Transporteinheit 44 kann der Endlosschlauch 22 von der Materialrolle 20 abgewickelt werden. Das Abwickeln des Endlosschlauchs 22 sollte dabei vorzugsweise mit gleichbleibender Spannung erfolgen.

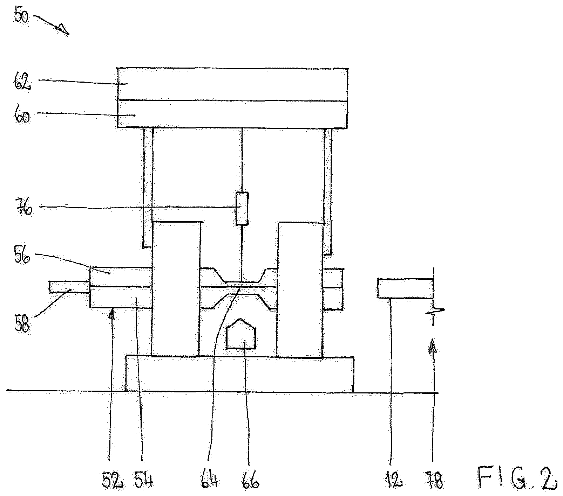

Das freie Ende 12 des Endlosschlauchs 22 wird mittels der Greifereinheit 40 gehalten. Mittels einer Linearbewegung (Doppelpfeil 46) kann das freie Ende 12 des Endlosschlauchs 22 in eine Bearbeitungsstation 50 eingeschoben werden (siehe insbesondere Fig. 2). Die Bearbeitungsstation 50 besitzt ein Formwerkzeug 52, das im vorliegenden Beispielsfall aus zwei Formteilen 54, 56 in Form von Halbschalen besteht. Im Gegensatz zu dem hier dargestellten Ausführungsbeispiel könnte das Formwerkzeug 52 auch einteilig ausgeführt sein. Alternativ dazu könnte das Formwerkzeug 52 auch mehr als zwei Formteile aufweisen. Das freie Ende 12 des Endlosschlauchs 22 wird dabei so weit in das Formwerkzeug 52 eingeschoben, bis eine definierte Position oder eine definierte Kraft erreicht ist. Bei dieser Bewegung wird das freie Ende 12 des Endlosschlauchs 22 auf einen Werkzeugkern 58 des Formwerkzeugs 52 aufgeschoben. Der Durchmesser des Werkzeugkerns 58 ist dabei an den Innendurchmesser des Endlosschlauchs 22 angepasst. Vorzugsweise kann der Werkzeugkern 58 als separates Bauteil ausgebildet sein, so dass der Werkzeugkern 58 unabhängig vom Formwerkzeug 52 ausgetauscht werden kann. Das Aufschieben des freien Endes 12 des Endlosschlauchs 22 auf den Werkzeugkern 58 verhindert, dass der Schlauchinnendurchmesser bei dem nachfolgenden Umform-Prozess zugeschmolzen wird.

Für das Einschieben des freien Endes 12 in das Formwerkzeug 52 sind die Prozessparameter, insbesondere die Maximalgeschwindigkeit, die Beschleunigung, die Verzögerung, die Zielposition und die aufgebrachte Kraft beim Einschieben, grundsätzlich frei einstellbar. Die Prozessparameter können dadurch rasch und einfach an unterschiedliche Materialien und Abmessungen des Endlosschlauchs 22 angepasst werden. Auch eine Anpassung an unterschiedliche Formwerkzeuge 52 kann auf diese Weise unproblematisch vorgenommen werden.

Die Bearbeitungsstation 50 besitzt darüber hinaus ein Heizsystem 60, über die das Formwerkzeug 52 direkt oder indirekt erwärmt werden kann. Die Erwärmung des Formwerkzeugs 52 erfolgt im vorliegenden Beispielsfall durch einen kontinuierlich geregelten Strom durch das Formwerkzeug 52. Das Heizsystem 60 besitzt eine elektronische Temperaturregelung 62, so dass die Heizprozessparameter, wie insbesondere die vorgegebenen Temperaturen, die Aufheizrampe, die Abkühlrampe, die Zeit, über die die einzelnen Temperaturen gehalten werden sowie weitere Heizungsregelparameter, frei eingestellt werden können. Das Aufheizen des Formwerkzeugs 52 erfolgt gemäß dem Widerstandheizprinzip im Einstichbereich 64 des Formwerkzeugs 52. Dieser Einstichbereich 64 liegt im Bereich des kleinsten Querschnitts des Formwerkzeugs 52. Durch eine Anpassung der Geometrie des Formwerkzeugs 52, insbesondere hinsichtlich des Einstichbereichs 64 und der Querschnitte des Formwerkzeugs 52, kann die Erhitzungszone im Formwerkzeug 52 variiert und an unterschiedliche Gegebenheiten angepasst werden.

Nach dem Aktivieren des Heizsystems 60 wird das freie Ende 12 des Endlosschlauchs 22 nach einer definierten Zeit weiter in das Formwerkzeug 52 eingeschoben. Dabei wird die Außenkontur 70 des freien Endes 12 des Endlosschlauchs 22 umgeformt. Die Außenkontur 70 passt sich während des UmformProzesses an die Innenkontur 72 des Formwerkzeugs 52 an. Die Außenkontur 70 des verformten freien Endes 12 des Endlosschlauchs 22 wird somit durch die Innenkontur 72 des Formwerkzeugs 52 definiert. Die Innenkontur 72 des Formwerkzeugs 52 kann dabei nach Spezifikation gefertigt werden, so dass grundsätzlich beliebige Außenkonturen 70 realisiert werden können. Der Innendurchmesser des freien Endes 12 des Formwerkzeugs 52 wird durch die Auswahl des Werkzeugkerns 58 definiert. Sofern der Außendurchmesser des Werkzeugkerns 58 etwas geringer ist als der Innendurchmesser des freien Endes 12 des Endlosschlauchs 22, wird der Innendurchmesser des Endlosschlauchs 22 entsprechend reduziert. Die Länge dieser Reduzierung des Innendurchmessers kann durch die Positionierung des Werkzeugkerns 58 innerhalb des freien Endes 12 bestimmt werden.

Das Einschieben des freien Endes 12 während des Umform-Prozesses kann dabei nach definierten Prozessparametern erfolgen. Bei diesen Prozessparametern kann es sich beispielsweise um die Beschleunigung, die Verzögerung und/oder die Vorschubkraft handeln. Diese Prozessparameter können abhängig von dem Material und den Abmessungen des Endlosschlauchs 22 frei gewählt und damit an die jeweiligen Gegebenheiten angepasst werden.

Das Ende des Umform-Prozesses kann durch den zurückgelegten Weg der Transporteinheit 44 und/oder durch die Kraft beim Einschieben des Endlosschlauchs 22 in das Formwerkzeug 52 und/oder durch die aufgebrachte Zeit für den Umform-Prozess bestimmt werden. Nach dem Ende des Umform-Prozesses wird das Heizsystem 60 abgeschaltet, so dass das Formwerkzeug 52 abkühlt. Um das Abkühlen des Formwerkzeugs 52 zu beschleunigen, ist im vorliegenden Beispielsfall eine Kühlung 66 vorhanden. Diese Kühlung 66 kann nach dem Ende des Umform-Prozesses aktiviert werden. Das freie Ende 12 des Endlosschlauchs 22 verbleibt während des Abkühlens des Formwerkzeugs 52 in der eingefahrenen Position 74, damit das umgeformte freie Ende 12 des Endlosschlauchs 22 nicht versehentlich beschädigt oder erneut umgeformt wird. Diese eingefahrene Position 74 kann beispielsweise durch eine zurückgelegte Wegstrecke und/oder durch eine definierte Vorschubkraft bestimmt werden.

Die Temperatur des Formwerkzeugs 52 kann über eine Temperaturmessung 76 jederzeit erfasst, aufgezeichnet und ausgewertet werden. Die Temperaturmessung 76 kann dabei beispielsweise eine Widerstandsmessung, ein Thermoelement oder ein Pyrometer umfassen. Durch die Temperaturmessung 76 und die Temperaturregelung 62 ist darüber hinaus jederzeit eine präzise Anpassung der Heizprozessparameter möglich, sollten die gewünschten Temperaturen oder Temperaturverläufe nicht eingehalten werden. Dadurch kann rasch auf Störungen im Umform-Prozess reagiert werden, so dass die Herstellung von fehlerhaften Schlecht-Teilen reduziert werden kann.

Nach dem Unterschreiten einer definierten Temperatur und/oder nach Ablauf einer definierten Zeitspanne wird die Kühlung abgeschaltet. Anschließend wird das Formwerkzeug 52 geöffnet. Dazu wird das bewegliche Formteil 56 in einer Linearbewegung in radialer Richtung von dem stationären Formteil 54 wegbewegt. Das verformte freie Ende 12 des Endlosschlauchs 22 verbleibt in dem stationären Formteil 54, so dass das freie Ende 12 durch das stationäre Formteil 54 weiterhin stabilisiert wird. Die Linearbewegung des beweglichen Formteils 56 kann dabei insbesondere pneumatisch oder elektrisch angetrieben sein. Um das Lösen des beweglichen Formteils 56 von dem verformten freien Ende 12 des Endlosschlauchs 22 zu erleichtern, kann das Formwerkzeug 52 zur Unterstützung des Entformungsprozesses während des Öffnens über einen definierten und geregelten Heizprozess nochmals erwärmt werden.

Nach dem Öffnen des Formwerkzeugs 52 wird das freie Ende 12 des Endlosschlauchs 22 mittels der Transporteinheit 44 aus dem stationären Formteil 54 herausgefahren, bis sich das freie Ende 12 in einer Entnahmeposition 78 befindet. In dieser Entnahmeposition 78 kann das freie Ende 12 an eine hier nicht näher dargestellte Entnahmevorrichtung übergeben werden. Bei einer solchen Entnahmevorrichtung kann es sich beispielsweise um einen Roboterarm oder um einen XY-Greifer handeln.

Im vorliegenden Beispielsfall wird der Endlosschlauch 22 vor oder nach der Übergabe an die Entnahmevorrichtung 78 abgelängt. Die Bearbeitungsstation 50 besitzt dazu eine Schneideinrichtung 80. Mittels der Schneideinrichtung 80 kann der Endlosschlauch 22 in Schlauchabschnitte 14 beliebiger Länge geschnitten werden. Die Länge des Schlauchabschnitts 14 wird dabei insbesondere durch die Position der Transporteinheit 44 bestimmt. Das Ablängen erfolgt in der Regel nach dem Umformen des freien Endes 12 des Endlosschlauchs 22, um eine zusätzliche Stabilisierung zu ermöglichen. Theoretisch wäre es jedoch auch möglich, das Ablängen des Endlosschlauchs 22 bereits vor dem Umformen vorzunehmen. Zum Ablängen des Endlosschlauchs 22 wird die stationäre Greifereinheit 34 geschlossen, so dass das nach dem Ablängen entstehende neue freie Ende 12 des Endlosschlauchs 22 lagefixiert und keine erneute Ausrichtung des freien Endes 12 des Endlosschlauchs 22 erforderlich ist.

Nach dem Ablängen kann das fertige Produkt von der Entnahmevorrichtung an eine weitere Bearbeitungsstation übergeben werden. Dabei kann es sich beispielsweise auch um eine weitere erfindungsgemäße Vorrichtung 10 handeln, so dass das gegenüberliegende Ende des Schlauchabschnitts 14 ebenfalls umgeformt werden kann.

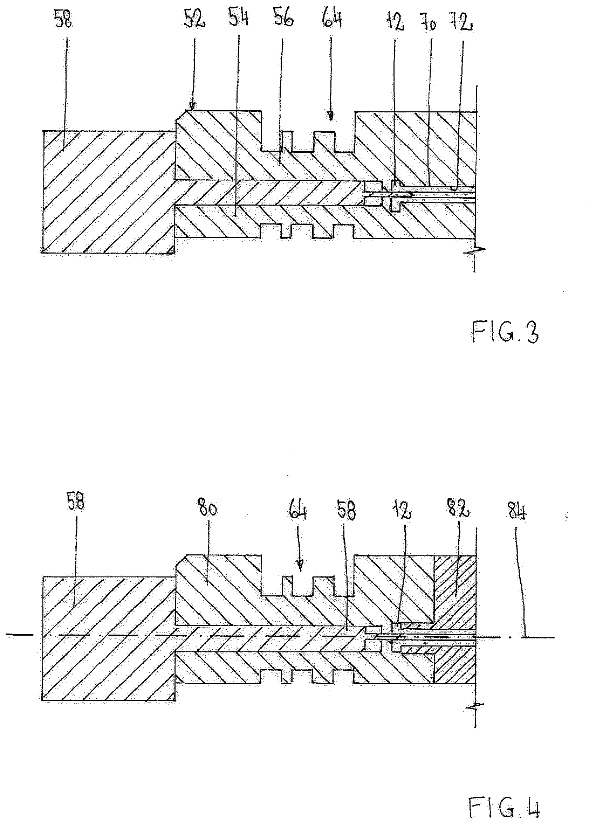

In Fig. 4 ist eine zweite Ausführungsform des Formwerkzeugs 52.4 dargestellt. Ein solches Formwerkzeug 52.4 kann wie das Formwerkzeug 52 gemäß Fig. 3 in der Bearbeitungsstation 50 einer erfindungsgemäßen Vorrichtung 10 eingesetzt werden.

Das Formwerkzeug 52.4 besitzt im vorliegenden Beispielsfall zwei hülsenartige Formteile 80, 82. Die beiden Formteile 80, 82 können in Längsrichtung 84 ein Stück weit ineinandergeschoben werden. Das freie Ende 12 des Endlosschlauchs 22 wird zum Umformen auf den Werkzeugkern 58 des Formwerkzeugs 52.4 aufgeschoben, wie dies oben bereits beschrieben ist. Der Durchmesser des Werkzeugkerns 58 ist dabei an den Innendurchmesser des Endlosschlauchs 22 angepasst. Vorzugsweise kann der Werkzeugkern 58 als separates Bauteil ausgebildet sein, so dass der Werkzeugkern 58 unabhängig vom Formwerkzeug 52.4 ausgetauscht werden kann.

Nach dem Aktivieren des Heizsystems 60 wird das freie Ende 12 des Endlosschlauchs 22 nach einer definierten Zeit weiter in das Formwerkzeug 52.4 eingeschoben. Dabei wird die Außenkontur 70 des freien Endes 12 des Endlosschlauchs 22 umgeformt. Die Außenkontur 70 passt sich während des Umform-Prozesses an die Innenkontur 72 des Formwerkzeugs 52.4 an. Die Außenkontur 70 des verformten freien Endes 12 des Endlosschlauchs 22 wird somit durch die Innenkontur 72 des Formwerkzeugs 52.4 definiert. Der Innendurchmesser des freien Endes 12 des Formwerkzeugs 52 wird durch die Auswahl des Werkzeugkerns 58 definiert.

Nach dem Ende des Umformprozesses wird das Formwerkzeug 52.4 geöffnet.

Dazu wird das bewegliche Formteil 82 in einer Linearbewegung in Längsrichtung aus dem stationären Formteil 80 herausbewegt. Das verformte freie Ende 12 des Endlosschlauchs 22 verbleibt in seiner Umformposition im Bereich des stationären Formteils 80, so dass das freie Ende 12 durch das stationäre Formteil 80 weiterhin stabilisiert wird. Die Linearbewegung des beweglichen Formteils 82 kann insbesondere pneumatisch oder elektrisch angetrieben sein. Um das Lösen des beweglichen Formteils 80 von dem verformten freien Ende 12 des Endlosschlauchs 22 zu erleichtern, kann das Formwerkzeug 52.4 während des Öffnens zur Unterstützung des Entformungsprozesses über einen definierten und geregelten Heizprozess nochmals erwärmt werden.

Bei den vorliegenden Ausführungsformen handelt es sich um einen vollautomatischen Prozess, bei dem sowohl die Zuführung des Endlosschlauchs 22 als auch die Entnahme des fertig umgeformten Produkts automatisiert erfolgt.

Alternativ dazu sind auch halbautomatische Prozesse möglich. In diesen Fällen könnten beispielsweise statt des Endlosschlauchs 22 bereits fertig abgelängte Schlauchabschnitte 14 zugeführt werden. Das Fixieren der Schlauchabschnitt in der Transporteinheit 44 kann in diesem Fall automatisiert oder manuell über ein händisches Einlegen der Schlauchabschnitte in die Greifereinheit 40 erfolgen.

Die Entnahme des fertigen Produkts kann bei halbautomatischen Prozessen teilautomatisiert oder manuell erfolgen. So könnte beispielsweise eine manuelle Entnahme der fertigen Produkte aus dem stationären Formteil 54 erfolgen.

## Patentansprüche

1. Vorrichtung (10) zum Formen des freien Endes (12) eines Schlauchabschnitts (14, 22),
- mit einer Zuführeinheit für den Schlauchabschnitt (14, 22),
- mit einer Transporteinheit (44) für den Schlauchabschnitt (14, 22),
- mit zumindest einer Bearbeitungsstation (50) für das freie Ende (12) des Schlauchabschnitts (14, 22),
- **dadurch gekennzeichnet, dass**
- die Bearbeitungsstation (50) einen Werkzeugkern (58) aufweist, über den das freie Ende (12) des Schlauchabschnitts (14, 22) schiebbar ist,
- die Bearbeitungsstation (50) ein Formwerkzeug (52, 52.4) aufweist, das von außen um das freie Ende (12) des Schlauchabschnitts (14, 22) herum positionierbar ist, so dass die Außenkontur (70) des freien Endes (12) des Schlauchabschnitts (14, 22) durch das Formwerkzeug (52, 52.4) verformbar ist,
- die Bearbeitungsstation (50) ein Heizsystem (60) aufweist, mittels dem das freie Ende (12) des Schlauchabschnitts (14, 22) und/oder das Formwerkzeug (52, 52.4) erwärmbar ist.

2. Vorrichtung nach Anspruch 1,
- **dadurch gekennzeichnet, dass**
- die Transporteinheit (44) eine Linearbewegung (46) des Schlauchabschnitts (14, 22) innerhalb der Bearbeitungsstation (50), insbesondere innerhalb des Formwerkzeugs (52, 52.4), ermöglicht.

3. Vorrichtung nach Anspruch 2,
- **dadurch gekennzeichnet, dass**
- die Transporteinheit (44) eine Greifereinheit (40) aufweist, durch die der Schlauchabschnitt (14, 22) fixierbar ist,
- die Greifereinheit (40) auf einer Lineareinheit (42) befestigt ist, so dass die Greifereinheit (40) in einer Linearbewegung (46) hin und her verfahrbar ist.

4. Vorrichtung nach einem der vorstehenden Ansprüche,
- **dadurch gekennzeichnet, dass**
- zumindest eine Sensoreinheit vorhanden ist, mittels der eine Überprüfung des Schlauchabschnitts (14, 22) durchführbar ist.

5. Vorrichtung nach einem der vorstehenden Ansprüche,
- **dadurch gekennzeichnet, dass**
- das Heizsystem (60) nach dem Widerstandsheizverfahren arbeitet.

6. Vorrichtung nach einem der vorstehenden Ansprüche,
- **dadurch gekennzeichnet, dass**
- die Bearbeitungsstation (50) eine Kühlung (66) aufweist.

7. Vorrichtung nach einem der vorstehenden Ansprüche,
- **dadurch gekennzeichnet, dass**
- das Formwerkzeug (52, 52.4) zumindest zwei Formteile (54, 56, 80, 82) aufweist,
- die zumindest zwei Formteile (54, 56, 80, 82) insbesondere in radialer Richtung oder in Längsrichtung (84) aufeinander zu und voneinander weg bewegbar sind, um das Formwerkzeug (52, 52.4) zu schließen und zu öffnen.

8. Verfahren zum Formen des freien Endes eines Schlauchabschnitts mit folgenden Verfahrensschritten:
- das freie Ende (12) des Schlauchabschnitts (14, 22) wird über eine Linearbewegung (46) in eine Bearbeitungsstation (50) eingeführt, dabei wird das freie Ende (12) des Schlauchabschnitts (14, 22) über einen Werkzeugkern (58) geführt,
- das freie Ende (12) des Schlauchabschnitts (14, 22) und/oder das Formwerkzeug (52, 52.4) der Bearbeitungsstation (50) wird erhitzt,
- das freie Ende (12) des Schlauchabschnitts (14, 22) wird über eine Linearbewegung (46) weiter in das Formwerkzeug (52, 52.4) eingefahren, so dass eine Umformung der Außenkontur (70) des freien Endes (12) des Schlauchabschnitts (14, 22) erfolgt.

9. Verfahren nach Anspruch 8,
- **dadurch gekennzeichnet, dass**
- der Schlauchabschnitt (14, 22) vor dem Einführen in die Bearbeitungsstation (50) mittels zumindest einer Sensoreinheit überprüft und/oder mittels einer vorgelagerten Bearbeitungsstation (30, 32) vorbehandelt wird.

10. Verfahren nach Anspruch 8 oder 9,
- **dadurch gekennzeichnet, dass**
- das Erhitzen des Formwerkzeugs (52, 52.4) der Bearbeitungsstation (50) nach Beendigung des Umform-Prozesses beendet wird,
- das freie Ende (12) des Schlauchabschnitts (14, 22) nach Beendigung des Umform-Prozesses und dem Ende des Erhitzens des Formwerkzeugs (52, 52.4) für eine gewisse Zeit in der eingefahrenen Position (74) verbleibt.

11. Verfahren nach Anspruch 10,
- **dadurch gekennzeichnet, dass**
- nach dem Ende des Erhitzens des Formwerkzeugs (52, 52.4) eine Kühlung (66) aktiviert wird.

12. Verfahren nach einem der Ansprüche 8 bis 11,
- **dadurch gekennzeichnet, dass**
- das Formwerkzeug (52, 52.4) vor dem weiteren Einfahren des freien Endes (12) des Schlauchabschnitts (14, 22) geschlossen wird,
- das Formwerkzeug (52, 52.4) nach dem Umformen der Außenkontur (70) des freien Endes (12) des Schlauchabschnitts (14, 22) geöffnet wird, um das Entnehmen des verformten Schlauchabschnitts (14, 22) zu erleichtern.

13. Verfahren nach Anspruch 12,
- **dadurch gekennzeichnet, dass**
- das Formwerkzeug (52, 52.4) während des Öffnens erwärmt wird.

14. Verfahren nach einem der Ansprüche 8 bis 13,
- **dadurch gekennzeichnet, dass**,
- das verformte freie Ende (12) des Schlauchabschnitts (14, 22) über eine Linearbewegung (46) aus dem Formwerkzeug (52, 52.4) herausgeführt wird.

15. Verfahren nach einem der Ansprüche 8 bis 14,
- **dadurch gekennzeichnet, dass**
- die Schlauchabschnitte (14) als Endlosschlauch (22) zugeführt werden,
- von dem Endlosschlauch (22) nach dem Umformen der Außenkontur (70) des freien Endes (12) ein Schlauchabschnitt (14) der gewünschten Länge abgeschnitten wird.
